# EUROPEAN PATENT SPECIFICATION

(11) **EP 1 254 104 B1**
(45) Date of publication and mention of the grant of the patent: **13.04.2005**
(21) Application number: 00986919.9
(22) Date of filing: 15.12.2000
(51) Int. Cl.: C07C 217/14, C07C 217/30, C07D 211/14, A61K 31/135, A61P 11/14

(54) **QUATERNARY AMMONIUM COMPOUNDS AND THEIR USE AS ANTI-TUSSIVE AGENT**
QUATERNÄRE AMMONIUMVERBINDUNGEN UND IHRE VERWENDUNG ALS MITTEL GEGEN HUSTEN
COMPOSES D'AMMONIUM QUATERNAIRE ET LEUR UTILISATION COMME AGENT ANTITUSSIF

(30) Priority: 15.12.1999 CA 2292343
(43) Date of publication of application: 06.11.2002
(73) Proprietor: UCB FARCHIM, S.A., 1630 Bulle (CH)
(72) Inventor: CHOI, Lewis, Siu, Leung, Burnaby, British Columbia V5A 3P8 (CA); BEATCH, Gregory, N., Vancouver, British Columbia V6S 1P5 (CA); PAGE, Clive, P., London, Greater London SW11 3JP (GB)
(74) Representative: Lechien, Monique
(86) International application number: PCT/CA2000/001508
(87) International publication number: WO 2001/044167

(56) References cited:
- WO-A-99/64398
- GB-A- 919 126
- GB-A- 935 613
- US-A- 2 642 434
- US-A- 2 703 324
- US-A- 3 840 666
- US-A- 4 048 335
- US-A- 4 327 214
- CHEMICAL ABSTRACTS, vol. 108, no. 15, 11 April 1988 (1988-04-11) Columbus, Ohio, US; abstract no. 131146y, STANKEVICIENE, L. ET AL.: "ynthesis of propanolamine derivatives and study of thgeir antiviral activity." page 691; column 2; XP002162720 & KHIM.-FARM. ZH., vol. 21, no. 3, 1987, pages 328-332,
- R. K. JOSHI ET AL.: "Note on the synthesis of some quaternary N-(omega-aryloxyalkyl) Piperidinium, Pyridinium, Benzyl-dimethyl-ammonium, and Trimethyl-ammonium bromides" HELVETICA CHIMICA ACTA., vol. 54, no. 9-10, 1971, pages 112-117, XP000982730 VERLAG HELVETICA CHIMICA ACTA. BASEL., CH ISSN: 0018-019X

## Description

### BACKGROUND OF THE INVENTION

Conventional cough preparations containing an effective anti-tussive agent such as codeine have long been used for the symptomatic relief of coughs. However, codeine has various side effects which are undesirable.

Accordingly, the present invention relates to compounds and pharmaceutical compositions having anti-tussive activity, and a method of treating and/or preventing coughs in warm-blooded animals in need thereof by administering an effective amount of the compounds or the pharmaceutical compositions of the invention.

### SUMMARY OF THE INVENTION

The problems of the prior art have been overcome by the present invention, which discloses compounds and pharmaceutical compositions possessing anti-tussive activity, and a method of administering the same to warm-blooded animals, including humans. The present invention is related to quaternary ammonium compounds that have been found to be useful in the treatment and/or prevention of cough.

In one aspect, the present invention concerns the use of certain quaternary ammonium compounds as active ingredient in the manufacture of a medicament for use in the treatment and/or prevention of cough in warm-blooded animals, including humans.

Chemical Abstracts, vol. 108, no. 15, abstract no. 131146y, discloses propanolamine derivatives. However, in this document, the compounds are only disclosed in the context of antiviral activity, hence, with a completely different therapeutic indication than cough treatment.

US 3,840,666 discloses the compound N,N-dimethyl-propranolol chloride as such and its use in the treatment of cardiac arrythmias. This document does neither disclose nor suggest the therapeutic indication cough.

US 919,126 discloses quaternary ammonium compounds, which are derivatives of N,N,N-trimethyl-mexiletine chloride, distinguished by containing an additional benzoyl group. These compounds have sympatholytic and anaesthetic activity.

WO 99/64398 forms state of the art within the meaning of Art. 54(3) EPC and may not be used in the assessment of inventive step (Art. 56 EPC). This document discloses quaternary ammonium salts, however, the specific compounds according to the claims of this invention are not disclosed.

Another aspect of the present invention discloses a method for the treatment and/or prevention of cough in warm-blooded animals, including humans, which method comprises administering to a warm-blooded animal in need thereof certain quaternary ammonium compounds.

Another aspect of the present invention discloses certain novel quaternary ammonium compounds that are useful for the treatment and/or prevention of cough in warm-blooded animals, including humans.

In another aspect, the present invention discloses a pharmaceutical composition for the treatment and/or prevention of cough, comprising an effective amount of certain novel quaternary ammonium compounds and a pharmaceutically acceptable carrier, diluent or excipient.

### BRIEF DESCRIPTION OF THE DRAWINGS

Figure 1 is a flow diagram showing the layout of the experimental apparatus used for cough determination; and
Figures 2A and 2B are expanded scale recordings of pressure changes derived from the differential pressure transducer during characteristic responses exhibited by a guinea-pig during exposure to an aerosol of citric acid.

### DETAILED DESCRIPTION OF THE INVENTION

As used herein, the following terms have the following meaning:
"Pharmaceutically acceptable carriers" for therapeutic use are well known in the pharmaceutical art, and are described, for example, in Remingtons Pharmaceutical Sciences, Mack Publishing Co. (A.R Gennaro edit. 1985). For example, sterile saline and phosphate-buffered saline at physiological pH may be used. Preservatives, stabilizers, dyes and even flavoring agents may be provided in the pharmaceutical composition. For example, sodium benzoate, sorbic acid and esters of *p*-hydroxybenzoic acid may be added as preservatives. In addition, antioxidants and suspending agents may be used.
"Pharmaceutically acceptable salt" refers to salts of the compounds of the present invention derived from the combination of such compounds and an organic or inorganic acid (acid addition salts) or an organic or inorganic base (base addition salts). The compounds of the present invention may be used in either the free base or salt forms, with both forms being considered as being within the scope of the present invention.

The "therapeutically effective amount" of a compound of the present invention will depend on the route of administration, the type of warm-blooded animal being treated, and the physical characteristics of the specific warm-blooded animal under consideration. These factors and their relationship to determining this amount are well known to skilled practitioners in the medical arts. This amount and the method of administration can be tailored to achieve optimal efficacy but will depend on such factors as weight, diet, concurrent medication and other factors which those skilled in the medical arts will recognize.

Compositions described herein as "containing a compound of formula (I)" encompass compositions that contain more than one compound of formula (I).

The origin of the cough to be treated by the present invention is not particularly limited, and can include virtually any respiratory disorder, such as chronic obstructive pulmonary disease, tuberculosis, bronchitis, respiratory malignancies, asthma, allergy, pulmonary fibrosis, respiratory tract inflammation, emphysema, pneumonia, lung cancer, presence of foreign bodies, soar throat, common cold, influenza, respiratory tract infection, bronchoconstriction, inhalation of irritants, smoker's cough, chronic non-productive cough, neoplastic cough, cough due to angiotension converting enzyme (ACE) inhibitor therapy, etc. Cough may also occur without a known cause.

This invention describes certain quaternary ammonium compounds and their utility as anti-tussive agents. The invention relates to the discovery that certain quaternary ammonium compounds and pharmaceutically acceptable salts, esters, amides, complexes, chelates, solvates, stereoisomers, stereoisomeric mixtures, geometric isomers, crystalline or amorphous forms, metabolites, metabolic precursors or prodrugs thereof, are useful in the treatment and/or prevention of cough in warm-blooded animals, including humans.

The present invention provides the use of a compound in the manufacture of a medicament for the treatment and/or prevention of cough in a warm-blooded animal, which method comprises administering to a warm-blooded animal in need thereof, a therapeutically effective amount of a compound of formula (I) which is N,N,N-trimethyl-mexiletine chloride having the following structure, or a pharmaceutically acceptable complex, chelate, solvate, stereoisomer, stereoisomeric mixture, crystalline or amorphous form, metabolite, metabolic precursor or prodrug thereof:

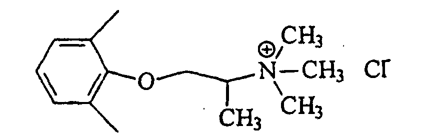

The present invention further provides the use of a compound in the manufacture of a medicament for the treatment and/or prevention of cough in a warm-blooded animal, which method comprises administering to a warm-blooded animal in need thereof, a therapeutically effective amount of a compound of formula (I) which is N,N-dimethyl-propranolol chloride having the following structure, or a pharmaceutically acceptable complex, chelate, solvate, stereoisomer, stereoisomeric mixture, crystalline or amorphous form, metabolite, metabolic precursor or prodrug thereof:

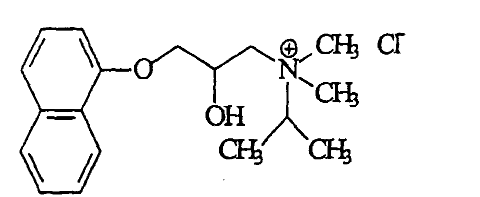

The present invention further provides the use of a compound in the manufacture of a medicament for the treatment and/or prevention of cough in a warm-blooded animal, which method comprises administering to a warm-blooded animal in need thereof, a therapeutically effective amount of a compound of formula (I) which is N,N-dimethyl-pindolol chloride having the following structure, or a pharmaceutically acceptable complex, chelate, solvate, stereoisomer, stereoisomeric mixture, crystalline or amorphous form, metabolite, metabolic precursor or prodrug thereof:

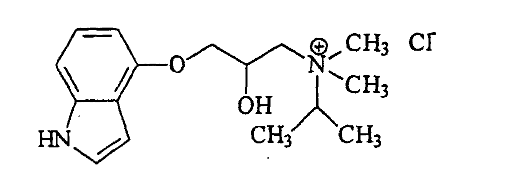

The present invention further provides for the use of a compound selected from N,N,N-trimethyl-mexiletine iodide, N,N-dimethyl-propranolol iodide and N,N-dimethyl-pindolol iodide as active ingredient in the manufacture of a medicament for use in the treatment and/or prevention of cough in a warm-blooded animal.

The compounds of the present invention may be prepared by direct quaternisation of the corresponding amino precursors with an appropriate alkyl halide. For example, N,N,N-trimethyl-mexiletine chloride is synthesized by treatment of mexiletine (commercially available from e.g. Sigma-Aldrich) with methyl chloride. Similarly N,N,N-trintethyl-mexiletine iodide is synthesized by treatment of mexiletine (commercially available from e.g. Sigma-Aldrich) with methyl iodide. Quaternary propranolol such as N,N-dimethyl-propranolol chloride and N,N-dimethyl-propranolol iodide can be similarly prepared from propranolol (commercially available from e.g. Sigma-Aldrich) and methyl chloride or methyl iodide respectively. Conditions for these preparations and other closely related analogs were described in e.g. U.S. 4,048,335. In an analogous approach, quaternary pindolol can be synthesized by treatment of pindolol (commercially available from e.g. Sigma-Aldrich) with the appropriate alkyl halide. N,N-dimethyl-pindolol chloride can thus be synthesized by reaction of pindolol with methyl chloride.

The synthetic procedures described herein, especially when taken with the general knowledge in the art, provide sufficient guidance to those of ordinary skill in the art to perform the synthesis, isolation, and purification of the compounds of the present invention.

It is recognized that there is at least one chiral center in the compounds used within the scope of the present invention and thus such compounds will exist as various stereoisomeric forms. Applicants intend to include all the various stereoisomers within the scope of the invention. Though the compounds may be prepared as racemates and can conveniently be used as such, individual enantiomers also can be isolated or preferentially synthesized by known techniques if desired. Such racemates and individual enantiomers and mixtures thereof are intended to be included within the scope of the present invention. Pure enantiomeric forms if produced may be isolated by preparative chiral HPLC. The free base may be converted if desired, to the monohydrochloride salt by known methodologies, and subsequently, if desired, to other acid addition salts by reaction with inorganic or organic salts. Acid addition salts can also be prepared metathetically by reacting one acid addition salt with an acid that is stronger than that of the anion of the initial salt.

The present invention also encompasses the pharmaceutically acceptable salts, esters, amides, complexes, chelates, solvates, crystalline or amorphous forms, metabolites, metabolic precursors or prodrugs of the compounds according to claims 1 to 6. Pharmaceutically acceptable esters and amides can be prepared by reacting, respectively, a hydroxy or amino functional group with a pharmaceutically acceptable organic acid, such as identified below. A prodrug is a drug which has been chemically modified and may be biologically inactive at its site of action, but which is degraded or modified by one or more enzymatic or other in vivo processes to the parent bioactive form. Generally, a prodrug has a different pharmakokinetic profile than the parent drug such that, for example, it is more easily absorbed across the mucosal epithelium, it has better salt formation or solubility and/or it has better systemic stability (e. g., an increased plasma half-life).

Those skilled in the art recognize that chemical modifications of a parent drug to yield a prodrug include: (1) terminal ester or amide derivatives which are susceptible to being cleaved by esterases or lipases; (2) terminal peptides which may be recognized by specific or nonspecific proteases; or (3) a derivative that causes the prodrug to accumulate at a site of action through membrane selection, and combinations of the above techniques. Conventional procedures for the selection and preparation of prodrug derivatives are described in H. Bundgaard, Design of Prodrugs, (1985). Those skilled in the art are well-versed in the preparation of prodrugs and are well-aware of its meaning.

In another embodiment, the present invention provides compositions which include a compound of the present invention as described above in admixture or otherwise in association with one or more inert carriers, excipients and diluents, as well as optional ingredients if desired. Inert carriers include any material which does not degrade or otherwise covalently react with a compound of the invention. Thus, the present invention provides a pharmaceutical or veterinary composition (hereinafter, simply referred to as a pharmaceutical composition) containing a compound of the present invention as described above, in admixture with a pharmaceutically acceptable carrier, excipient or diluent. The invention further provides a pharmaceutical composition containing an effective amount of a compound of the present invention as described above, in association with a pharmaceutically acceptable carrier.

The pharmaceutical compositions of the present invention may be in any form which allows for the composition to be administered to a patient. For example, the composition may be in the form of a solid, liquid or gas (aerosol). Typical routes of administration include, without limitation, oral, topical, parenteral, sublingual, rectal, vaginal, and intranasal. The term parenteral as used herein includes subcutaneous injections, intravenous, intramuscular, epidural, intrastemal injection or infusion techniques. Pharmaceutical composition of the invention are formulated so as to allow the active ingredients contained therein to be bioavailable for administration of the composition to a patient. Compositions that will be administered to a patient take the form of one or more dosage units, where for example, a tablet, capsule or cachet may be a single dosage unit, and a container of a compound of the present invention in aerosol form may hold a plurality of dosage units.

Materials used in preparing the pharmaceutical compositions should be pharmaceutically pure and non-toxic in the amounts used. The inventive compositions may include one or more compounds (active ingredients) known for a particularly desirable effect. It will be evident to those of ordinary skill in the art that the optimal dosage of the active ingredient(s) in the pharmaceutical composition will depend on a variety of factors. Relevant factors include, without limitation, the type of subject (*e.g.*, human), the particular form of the active ingredient, the manner of administration and the composition employed.

In general, the pharmaceutical composition includes a compound of the present invention as described herein, in admixture with one or more carriers. The carrier(s) may be particulate, so that the compositions are, for example, in tablet or powder form. The carrier(s) may be liquid, with the compositions being, for example, an oral syrup or injectable liquid. In addition, the carrier(s) may be gaseous, so as to provide an aerosol composition useful in, *e.g.*, inhalatory administration.

When intended for oral administration, the composition is preferably in either solid or liquid form, where semi-solid, semi-liquid, suspension and gel forms are included within the forms considered herein as either solid or liquid.

As a solid composition for oral administration, the composition may be formulated into a powder, granule, compressed tablet, pill, capsule, cachet, chewing gum, wafer, lozenges, or the like form. Such a solid composition will typically contain one or more inert diluents or edible carriers. In addition, one or more of the following adjuvants may be present: binders such as syrups, acacia, sorbitol, polyvinylpyrrolidone, carboxymethylcellulose, ethyl cellulose, microcrystalline cellulose, gum tragacanth or gelatin, and mixtures thereof; excipients such as starch, lactose or dextrins, disintegrating agents such as alginic acid, sodium alginate, Primogel, corn starch and the like; lubricants such as magnesium stearate or Sterotex; fillers such as lactose, mannitols, starch, calcium phosphate, sorbitol, methylcellulose, and mixtures thereof, lubricants such as magnesium stearate, high molecular weight polymers such as polyethylene glycol, high molecular weight fatty acids such as stearic acid, silica, wetting agents such as sodium lauryl sulfate, glidants such as colloidal silicon dioxide; sweetening agents such as sucrose or saccharin, a flavoring agent such as peppermint, methyl salicylate or orange flavoring, and a coloring agent.

When the composition is in the form of a capsule, *e.g.*, a gelatin capsule, it may contain, in addition to materials of the above type, a liquid carrier such as polyethylene glycol or a fatty oil.

The composition may be in the form of a liquid, *e.g.*, an elixir, syrup, solution, aqueous or oily emulsion or suspension, or even dry powders which may be reconstituted with water and/or other liquid media prior to use. The liquid may be for oral administration or for delivery by injection, as two examples. When intended for oral administration, preferred compositions contain, in addition to the present compounds, one or more of a sweetening agent, thickening agent, preservative (*e.g.,* alkyl *p*-hydoxybenzoate), dye/colorant and flavor enhancer (flavorant). In a composition intended to be administered by injection, one or more of a surfactant, preservative (*e.g.*, alkyl *p*-hydroxybenzoate), wetting agent, dispersing agent, suspending agent (*e.g.,* sorbitol, glucose, or other sugar syrups), buffer, stabilizer and isotonic agent may be included. The emulsifying agent may be selected from lecithin or sorbitol monooleate.

The liquid pharmaceutical compositions of the invention, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or digylcerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

A liquid compositions intended for either parenteral or oral administration should contain an amount of the inventive compound such that a suitable dosage will be obtained. Typically, this amount is at least 0.01% of a compound of the invention in the composition. When intended for oral administration, this amount may be varied to be between 0.1 and about 70% of the weight of the composition. Preferred oral compositions contain between about 4% and about 50% of the active compound of the present invention. Preferred compositions and preparations according to the present invention are prepared so that a parenteral dosage unit contains between 0.01 to 10% by weight of active compound.

The pharmaceutical composition may be intended for topical administration, in which case the carrier may suitably comprise a solution, emulsion, ointment, cream or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device. Topical formulations may contain a concentration of the inventive compound of from about 0.1 to about 25% w/v (weight per unit volume).

The composition may be intended for rectal administration, in the form, *e.g.,* of a suppository which will melt in the rectum and release the drug. The composition for rectal administration may contain an oleaginous base as a suitable nonirritating excipient. Such bases include, without limitation, lanolin, cocoa butter and polyethylene glycol. Low-melting waxes are preferred for the preparation of a suppository, where mixtures of fatty acid glycerides and/or cocoa butter are suitable waxes. The waxes may be melted, and the compound of the present invention is dispersed homogeneously therein by stirring. The molten homogeneous mixture is then poured into convenient sized molds, allowed to cool and thereby solidify.

The composition may include various materials which modify the physical form of a solid or liquid dosage unit. For example, the composition may include materials that form a coating shell around the active ingredients. The materials which form the coating shell are typically inert, and may be selected from, for example, sugar, shellac, and other enteric coating agents. Alternatively, the active ingredients may be encased in a gelatin capsule or cachet.

The composition in solid or liquid form may include an agent which binds to the compound of the present invention and thereby assists in the delivery of the active components. Suitable agents which may act in this capacity include a monoclonal or polyclonal antibody, a protein or a liposome.

The pharmaceutical composition of the present invention may consist of gaseous dosage units, *e.g.*, it may be in the form of an aerosol. The term aerosol is used to denote a variety of systems ranging from those of colloidal nature to systems consisting of pressurized packages. Delivery may be by a liquefied or compressed gas or by a suitable pump system which dispenses the active ingredients. Aerosols of compounds of the invention may be delivered in single phase, bi-phasic, or tri-phasic systems in order to deliver the active ingredient(s). Delivery of the aerosol includes the necessary container, activators, valves, subcontainers, and the like, which together may form a kit. Preferred aerosols may be determined by one skilled in the art, without undue experimentation. The pharmaceutical compositions may be prepared by methodology well known in the pharmaceutical art. The compounds of the present invention may be in the form of a solvate in a pharmaceutically acceptable solvent such as water or physiological saline.

Suitable pharmaceutically acceptable salts include acid addition salts of acids such as hydrochloric, hydrobromic, benzenesulfonic (besylate), benzoic, camphorsulfonic, ethanesulfonic, fumaric, gluconic, glutamic, isethionic, maleic, malic, mandelic, methanesulfonic, mucic, nitric, pamoic, pantothenic, succinic, p-toluenesulfonic, phosphoric, sulphuric, citric, tartaric, lactic and acetic acid, although the preferred acid addition salt is the hydrochloride salt.

As indicated above, the magnitude of the therapeutic or prophylactic dose of the compounds of the present invention in the treatment and/or prevention of cough will depend upon the severity and nature of the condition being treated and the route of administration. The dose and the frequency of the dosing will also vary according to age, body weight and response of the individual patient. In general, the total daily dose range for the compounds of the present invention for the treatment or prevention of cough is from about 0.1 to about 800 mg in single or repeated doses.

Any suitable route of administration as described above may be employed to provide an effective dosage of the compounds of the present invention, although administration by inhalation is preferred, most preferably in aerosol form. Suitable forms of administration include, but are not limited to, inhalation (delivered by, e.g., metered-dose inhaler, jet nebulizer, ultrasonic nebulizer, dry powder inhaler, etc.), nasal sprays, nebulization, oral administration such as via tablets, capsules, lozenges, syrups, sprays, suspensions, elixirs, gargles, and other liquid preparations, aerosol foams, parental administration, and sublingal administration.

The compounds of the present invention can include pharmaceutically acceptable carriers and other conventional additives, including aqueous based carriers, co-solvents such as ethyl alcohol, propylene glycol and glycerin, fillers, lubricants, wetting agents, flavoring agents, coloring agents, emulsifying, suspending or dispersing agents, suspending agents, etc. For aerosol delivery of the compounds of the present invention, pharmaceutically acceptable diluents, carriers, and/or propellants may be included in the formulations for use in appropriate devices. These are prepared by procedures well known to those skilled in the art (see e.g. Medication Teaching Manual, 5th Ed., Bethesda, MD, American Society of Hospital Pharmacists, 1991).

The compositions of the present invention may optionally include other known therapeutic agents, including decongestants such as pseudoephedrine HCl, phenylephrine HCl and ephedrine HCl, non-steroidal anti-inflammatory drugs such as acetaminophen, aspirin, phenacetin, ibuprofen and ketoprofen, expectorants such as glyceryl guaiacolate, terpin hydrate and ammonium chloride, antihistamines such as chlorpheniramine maleate, doxylamine succinate, brompheniramine maleate and diphenhydramine hydrochloride, and anesthetic compounds such as phenol.

The following examples are offered by way of illustration and not by way of limitation.

### EXAMPLE 1

### Synthesis of 1-(2,6-Dimethyl phenoxy)-N,N,N-trimethylpropan-2-aminium iodide (N,N,N-trimethyl-mexiletine iodide)

Mexiletine hydrochloride (1.03 g, 4.76 mmol) was partioned between dichloromethane (20 mL) and saturated aqueous NaHCO₃ (5 mL). The aqueous layer was collected and extracted with dichloromethane (2 x 5 mL). The organic layers were combined and dried over anhydrous Na₂SO₄. To the filtrate was added methyl iodide (2.1 mL, 34.3 mmol) and Na₂CO₃ (1.61 g, 15.2 mmol). The reaction mixture was stirred at room temperature for 162 hours, filtered, and the filtrate was concentrated *in vacuo* to give a brown solid. Recrystallization from a mixture of ethanol and ether (1:4, v/v, 25 mL) gave the product as yellow crystals (1.28 g, 77% yield). ¹³C NMR (75 MHz, DMSO-d₆)δ 12.1 (CH₃), 16.4 (CH₃), 51.4 (CH₃), 68.9 (CH), 69.3 (CH₂), 124.4 (CH), 129.0 (CH), 130.3 (C), 154.6 (C).

### EXAMPLE 2

### Synthesis of 2-Hvdroxy-N-isopropyl-N,N-dimethyl-3-(1-naphthyloxy)propan-1-aminium iodide (N,N-dimethyl-propranolol iodide)

Propanolol hydrochloride (2.81 g, 9.49 mmol) was dissolved in H₂O (15 mL), saturated aqueous NaHCO₃ (45 mL), and extracted with dichloromethane (3 x 60 mL). The organic layers were combined, dried over anhydrous Na₂SO₄ and concentrated *in vacuo* to 50 mL. To the residual solution was added methyl iodide (2.8 mL, 45.1 mmol) and K₂CO₃ (4.1 g, 29.7 mmol). The reaction mixture was stirred for 63 hours at room temperature and then refluxed for 67 hours. The solvent was then concentrated *in vacuo* to give a white solid which was dissolved in H₂O (220 mL). The solution was extracted with ether (3 x 220 mL) and again with dichloromethane (3 x 220 mL). The combined dichloromethane extracts were dried over anhydrous Na₂SO₄ and the solvent removed *in vacuo* to give the crude product. Recrystallization from a mixture of ethanol and ether (3:2, v/v, 25mL) provided the product as yellow crystals (1.2 g, 30% yield). ¹³C NMR (75MHz, CDCl₃) δ 16.8 (CH₃), 16.9 (CH₃), 49.3 (CH₃), 49.8 (CH₃), 65.7 (CH), 65.8 (CH₂), 68.2 (CH), 71.6 (CH₂), 106.3 (CH), 121.9 (CH), 122.8 (CH), 126.4 (CH), 126.6 (CH), 126.9 (CH), 127.5 (CH), 128.5(CH), 135.9 (C), 155.2 (C).

### EXAMPLE 3

### Synthesis of 2-Hydroxy-3-(1H-indol-4-yloxy)-N-isopropyl-N,N-dimethylpropan-1-aminium iodide (N,N-dimethyl-pindolol iodide)

To a solution of pindolol (0.280 g, 1.13 mmol) in THF (60 mL) was added-methyl iodide (0.71 mL, 11.32 mmol). The resultant solution was stirred for 6 hours then refluxed for 40 hours. The precipitate was collected and washed with THF (5 x 5 mL) to give the title compound (0.10 g). The filtrate was then concentrated *in vacuo* to 30 ml, methyl iodide (0.50 mL, 8.03 mmol) was added, and the mixture was refluxed for 16 hours. The resultant precipitate was collected and washed with THF (3 x 5 ml) to yield a second crop (0.02 g) (29% overall yield). ¹³C NMR (75MHz, DMSO ) δ 15.9 (CH₃), 16.1 (CH₃), 48.0 (CH₃), 48.3 (CH₃), 63.8 (CH), 64.2 (CH₂), 65.5 (CH), 69.8 (CH₂), 98.4 (CH), 100.0 (CH), 105.3 (CH), 118.2 (C), 121.6 (CH), 123.6 (CH), 137.2 (C), 151,3 (C).

### EXAMPLE 4

The following method is one of the general methods available to determine the antitussive activity of the compounds of the present invention.

Male albino Dunkin-Hartley strain guinea-pigs (weight 300-400g) can be obtained from various commercial suppliers.

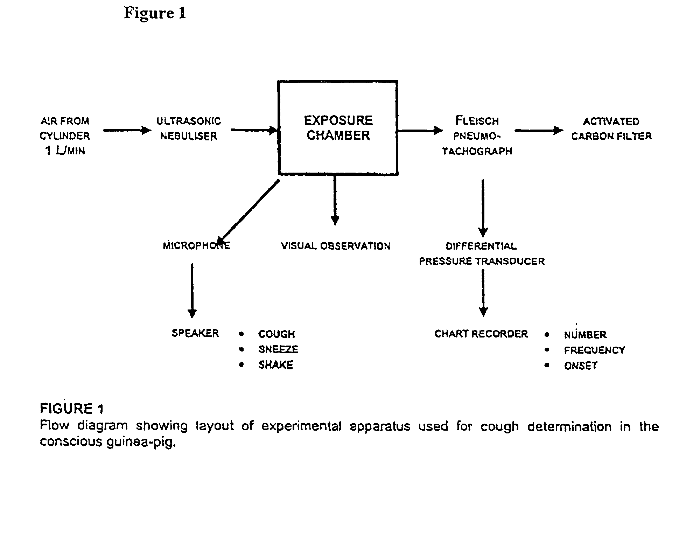

The method is a modification of that described by Adcock J.J., Schneider C. and Smith T.W., "Effects of Morphine and a Novel Opioid Pentapeptide BW443C, on Cough, Nociception and Ventilation in the Unanaesthetized Guinea-pig", *Br*. *J*. *Pharmacol*., **93,** 93-100 (1988). Individual conscious guinea-pigs are placed unrestrained into a sealed purpose built perspex exposure chamber (3,000 cm³ volume) and allowed to acclimatize prior to aerosol administration. The layout of the experimental apparatus used is shown in Figure 1.

Cylinder air is introduced into the exposure chamber at a flow rate of 1 liter/min, maintained by a needle valve and monitored by a rotameter. From the rotameter the air passes through the cup of an ultrasonic nebulizer (DeVilbis UltraNeb 2000) which is used to generate aerosols of drug or citric acid at 0.15 ml/min. A Fleisch pneumotachograph, connected to a differential pressure transducer (Grass model PT5) is attached to the outflow from the exposure chamber and provides a measurement of airflow from the chamber. The differential pressure transducer is connected to a Grass polygraph from which a hard copy record can be produced. The output from the polygraph is directed to a computerized data acquisition system (Poh-Ne-Mah) for real time recording of data. A tie-clip microphone is placed in the exposure chamber and connected via a pre-amplifier to a loudspeaker output to provide the observer with an audio monitor of responses.

Cough responses are induced by exposure to an aerosol of citric acid (1M) for 10 minutes. Animals are continuously monitored by trained observer, and the number of coughs are counted during a 15 minute period from commencement of the citric acid aerosol administration. Three characteristic responses can be produced by exposure to citric acid: cough, sneeze and "wet dog" shake.

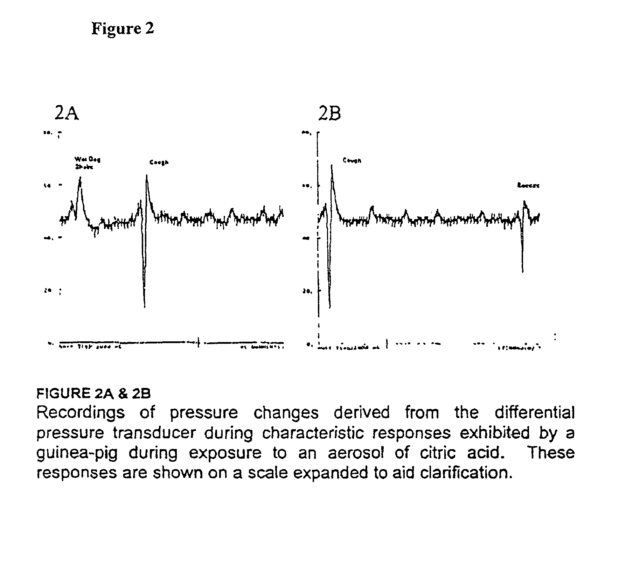

The three types of response are differentiated primarily by sound and visual observation. Confirmation of the numbers of multiple coughs is determined by reference to the change in flow rate displayed by the Poh-Ne-Mah system monitor. Printouts demonstrating the pressure changes characteristic of the different response to irritant are shown in Figures 2A and 2B. Data records for individual guinea-pigs on the Poh-Ne-Mah system are stored on an optical disk. Each cough is marked on the Grass polygraph paper trace, and from these record numbers, frequency and time of onset of coughs are determined. The cough response is defined by a characteristic coughing sound and behavior, associated with a marked biphasic pressure change. The biphasic pressure changes associated with a sneeze are not of as great a magnitude as those associated with a cough, the secondary rise in pressure also being far less than during a cough (Figure 2B). The sound of a sneeze differed from that of a cough, and sneezing is associated with nose rubbing activity. The third response, a "wet dog" shake, produces a rise in pressure only (Figure 2A) and lacked the definitive sound of a cough or sneeze.

Quantities of drugs are weighed out and dissolved in a vehicle. Equal volumes are aliquotted into sample tubes before being passed, together with another sample tube containing the same volume of vehicle, to an independent observer for coding. Pre-treatments are matched by concentration together with a vehicle control group. Two to five guinea-pigs are randomly allocated to each treatment group. Animals are pre-treated with either vehicle (e.g. distilled water, 0.9% sterile saline, Tween or 1 to 25% ethanol depending on the solubility of the compound), reference compound (e.g. mexiletine, propranolol or pindolol) or test drugs for 5 minutes immediately prior to citric acid aerosol exposure. Test drugs and reference compound are administered as aerosols at concentrations selected from 0.1,1.0, 2.0, 5.0 and 10.0 mg/ml. The sequence of pre-treatment administration is determined according to a 4x4 Latin Square design.

Data can be presented as the mean ±SEM number of coughs produced by individual guinea-pigs within each group during the 15 minute observation period or mean±SEM latency of cough and are analyzed using one way analysis of variance to compare mean responses between matched groups of animals (doses) and between unmatched groups (treatments) followed by the Tukey-Kramer multiple comparison test where appropriate.

In one set of experiments using the general protocol described above, the antitussive activity of N,N-dimethyl-propranolol iodide was tested. Results showed that pre-treatment of guinea pigs with aerosols of N,N-dimethyl-propranolol iodide at 10.0 mg/ml immediately before exposure to citric acid (1M) inhibited cough responses by 50-70% compared with vehicle (saline) pre-treated guinea pigs over the 15 minute observation period. Similarly, other quaternary ammonium compounds of the present invention can be evaluated by this method.

### EXAMPLE 5

In another experiment similar to that described above in Example 4, the duration of the antitussive effects of the compounds of the present invention against citric acid-induced cough responses can be investigated in conscious guinea pigs. Quaternary ammonium compounds of the present invention, reference compounds or vehicle are tested by administering as aerosol pre-treatments (0.1,1.0,2.0, 5.0 or 10.0 mg/ml, 5 minute duration) at 5 minutes, 30 minutes, 1 hour, 2 hours and 4 hours prior to induction of cough responses by citric acid aerosol. Data and results are analyzed as described in Example 4.

### EXAMPLE 6

The antitussive effects of a 5 minute pre-treatment with aerosolized compounds of the present invention and reference compound (e.g. mexiletine, propranolol or pindolol) on capsaicin aerosol-induced cough can be investigated in conscious guinea-pigs using a method similar to that described in Example 4. Data and results are analyzed as described in Example 4.

### EXAMPLE 7

Therapeutic treatment with the compounds of the present invention can also be determined by a similar method as described in Example 4. The antitussive effects of compounds of the present invention and reference compound (e.g. mexiletine, propranolol or pindolol) that are administered after induction of cough responses by exposure to citric acid aerosol are investigated in conscious guinea pigs. Vehicle or test agents are administered as aerosols (10, 5, 2, 1.0 or 0.1mg/ml; 5 minute duration) 2 minutes after exposure to citric acid aerosol began. Cough responses are recorded during a 15 minute observation period (t=0 to t=15 minutes) from initiation of the citric acid exposure. Data and results are analyzed as described in Example 4.

### EXAMPLE 8

### Investigation of antitussive activity of aerosolized test compound on citric acid-induced cough responses in conscious rabbits

### Protocol

Twenty-two male New Zealand white rabbits are randomly allocated to either of two groups of 11 rabbits.

Pairs of rabbits (control versus test) are placed in individual exposure chambers with an airflow of 5 liter/min through the chambers.

Each rabbit is exposed to ozone (3 ppm) for 1 hour.

The rabbits are then immediately exposed to aerosols of either vehicle (chamber 1) or test compound (10 mg/ml, chamber 2) at a nebulization rate of 0.9 ml/min.

Cough responses are induced with citric acid aerosol (1.6 M).

Coughs are counted during the 10 minute exposure to citric acid.

All rabbits are exposed to ozone before vehicle or test drug pre-treatment.

Data and results are analyzed as described in Example 4.

## Claims

1. Use of a compound in the manufacture of a medicament for the treatment and/or prevention of cough in a warm-blooded animal, wherein the compound is N,N,N-trimethyl-mexiletine chloride or a pharmaceutically acceptable complex, chelate, solvate, stereoisomer, stereoisomeric mixture, crystalline or amorphous form.

2. Use of a compound in the manufacture of a medicament for the treatment and/or prevention of cough in a warm-blooded animal, wherein the compound is N,N-dimethyl-propranolol chloride or a pharmaceutically acceptable complex, chelate, solvate, stereoisomer, stereoisomeric mixture, crystalline or amorphous form.

3. Use of a compound in the manufacture of a medicament for the treatment and/or prevention of cough in a warm-blooded animal, wherein the compound is N,N-dimethyl-pindolol chloride or a pharmaceutically acceptable complex, chelate, solvate, stereoisomer, stereoisomeric mixture, crystalline or amorphous form.

4. Use of a compound in the manufacture of a medicament for the treatment and/or prevention of cough in a warm-blooded animal, wherein the compound is N,N,N-trimethyl-mexiletine iodide.

5. Use of a compound in the manufacture of a medicament for the treatment and/or prevention of cough in a warm-blooded animal, wherein the compound is N,N-dimethyl-propranolol iodide.

6. Use of a compound in the manufacture of a medicament for the treatment and/or prevention of cough in a warm-blooded animal, wherein the compound is N,N-dimethyl-pindolol iodide.

## Patentansprüche

1. Verwendung einer Verbindung bei der Herstellung eines Arzneimittels für die Behandlung und/oder Prävention von Husten bei einem Warmblüter, wobei die Verbindung N,N,N-Trimethyl-mexiletinchlorid oder eine pharmazeutisch verträgliche Komplex-, Chelat-, Solvat-, Stereoisomer-, Stereoisomerengemisch-, kristalline oder amorphe Form ist.

2. Verwendung einer Verbindung bei der Herstellung eines Arzneimittels für die Behandlung und/oder Prävention von Husten bei einem Warmblüter, wobei die Verbindung N,N-Dime-thylpropranololchlorid oder eine pharmazeutisch verträgliche Komplex-, Chelat-, Solvat-, Stereoisomer-, Stereoisomerengemisch-, kristalline oder amorphe Form ist.

3. Verwendung einer Verbindung bei der Herstellung eines Arzneimittels für die Behandlung und/oder Prävention von Husten bei einem Warmblüter, wobei die Verbindung N,N-Dime-thyl-pindololchlorid oder eine pharmazeutisch verträgliche Komplex-, Chelat-, Solvat-, Stereoisomer-, Stereoisomerengemisch-, kristalline oder amorphe Form ist.

4. Verwendung einer Verbindung bei der Herstellung eines Arzneimittels für die Behandlung und/oder Prävention von Husten bei einem Warmblüter, wobei die Verbindung N,N,N-Trimethyl-mexiletinjodid ist.

5. Verwendung einer Verbindung bei der Herstellung eines Arzneimittels für die Behandlung und/oder Prävention von Husten bei einem Warmblüter, wobei die Verbindung N,N-Dime-thylpropranololjodid ist.

6. Verwendung einer Verbindung bei der Herstellung eines Arzneimittels für die Behandlung und/oder Prävention von Husten bei einem Warmblüter, wobei die Verbindung N,N-Dime-thyl-pindololjodid ist.

## Revendications

1. Utilisation d'un composé dans la fabrication d'un médicament pour le traitement et/ou la prévention de la toux chez un animal à sang chaud, dans laquelle le composé est le chlorure de N,N,N-triméthyl-mexilétine ou un complexe, chélate, solvate, stéréoisomère, mélange de stéréoisomères, une forme cristalline ou amorphe acceptables pharmaceutiquement.

2. Utilisation d'un composé dans la fabrication d'un médicament pour le traitement et/ou la prévention de la toux chez un animal à sang chaud, dans laquelle le composé est le chlorure de N,N-diméthylpropranolol ou un complexe, chélate, solvate, stéréoisomère, mélange de stéréoisomères, une forme cristalline ou amorphe acceptables pharmaceutiquement.

3. Utilisation d'un composé dans la fabrication d'un médicament pour le traitement et/ou la prévention de la toux chez un animal à sang chaud, dans laquelle le composé est le chlorure de N,N-diméthylpindolol ou un complexe, chélate, solvate, stéréoisomère, mélange de stéréoisomères, une forme cristalline ou amorphe acceptables pharmaceutiquement.

4. Utilisation d'un composé dans la fabrication d'un médicament pour le traitement et/ou la prévention de la toux chez un animal à sang chaud, dans laquelle le composé est le iodure de N,N,N-triméthyl-mexilétine.

5. Utilisation d'un composé dans la fabrication d'un médicament pour le traitement et/ou la prévention de la toux chez un animal à sang chaud, dans laquelle le composé est le iodure de N,N-diméthylpropranolol.

6. Utilisation d'un composé dans la fabrication d'un médicament pour le traitement et/ou la prévention de la toux chez un animal à sang chaud, dans laquelle le composé est le iodure de N,N-diméthylpindolof.
